# EUROPÄISCHE PATENTANMELDUNG

(11) **EP 0 646 381 A1**
(43) Veröffentlichungstag der Anmeldung: **05.04.1995**
(21) Anmeldenummer: 94114839.7
(22) Anmeldetag: 21.09.1994
(51) Int. Cl.: A61M 5/142, A61M 5/155, A61M 5/00

(54) **Infusionspumpe**

(30) Priorität: 04.10.1993 DE 4333736
(71) Anmelder: OPTON Feintechnik Kiel GmbH, D-24106 Kiel (DE)
(72) Erfinder: Baumann, Hans, Dr., D-24223 Raisdorf (DE)
(74) Vertreter: Biehl, Christian, Dipl.-Phys. Boehmert & Boehmert Anwaltssozietät

(57) **Zusammenfassung**

Die Erfindung betrifft eine Infusionspumpe (23) zur Implantation in den Körper eines Patienten zur medikamentösen Behandlung dieses Patienten. Die Einrichtung weist mindestens eine Einstichstelle (21,22) zum Auffüllen eines Vorratsbehälter zum Speichern eines gewählten Medikaments auf und besitzt eine flüssigkeitsfördernde Einrichtung zum Transport des Medikaments vom Vorratsbehälter. Desweiteren ist in der Einrichtung mindestens eine Vorrichtung zum begrenzten Einleiten des aus dem Vorratsbehälter kommenden, in diesem gespeicherten, ausgewählten Medikaments in den Körper vorhanden.

Dabei stellt sich die Erfindung die Aufgabe, das Auffüllen des Vorratsbehälters für das zu verabreichende Medikament durch die an dem Vorratsbehälter angebrachte Einstichstelle (21,22) sowie für einen Bolusseptum sicher und problemlos zu gestalten.

Dies wird dadurch erreicht, daß um oder an mindestens einer der vorgesehenen Einstichstellen (21,22) in die Infusionspumpe (23) mindestens eine extrakorporal detektierbare Einrichtung (24,25) angeordnet ist, so daß zur Injektion in diese Einstichstelle deren Lage extrakorporal bestimmt werden kann.

## Beschreibung

Die vorliegende Erfindung betrifft ein Infusionspumpe nach dem Oberbegriff des ersten Patentanspruchs.

Infusionspumpen, welche in den Körper eines Patienten implantiert werden, sind bereits bekannt und werden heute in vielen Bereichen der Medizin eingesetzt. Implantierbare Infusionspumpen, auch Medikamentenpumpen genannt, ermöglichen eine direkte, kontinuierliche Medikamentenverabreichung in das arterielle oder venöse System, sowie in den epidualen oder intraspiralen Raum des menschlichen Körpers. Dabei erlaubt diese Art der medikamentösen Versorgung eine so geringe Dosis (0.5 bis 1.5 ml/Tag), daß die Lebensqualität des Patienten weitgehend erhalten bleibt und belastende Nebenwirkungen auf den Organismus erheblich gemildert werden, wie sie bei herkömmlichen Therapien bekannt sind (Tabletten, Tropfen, Injektion, usw.).

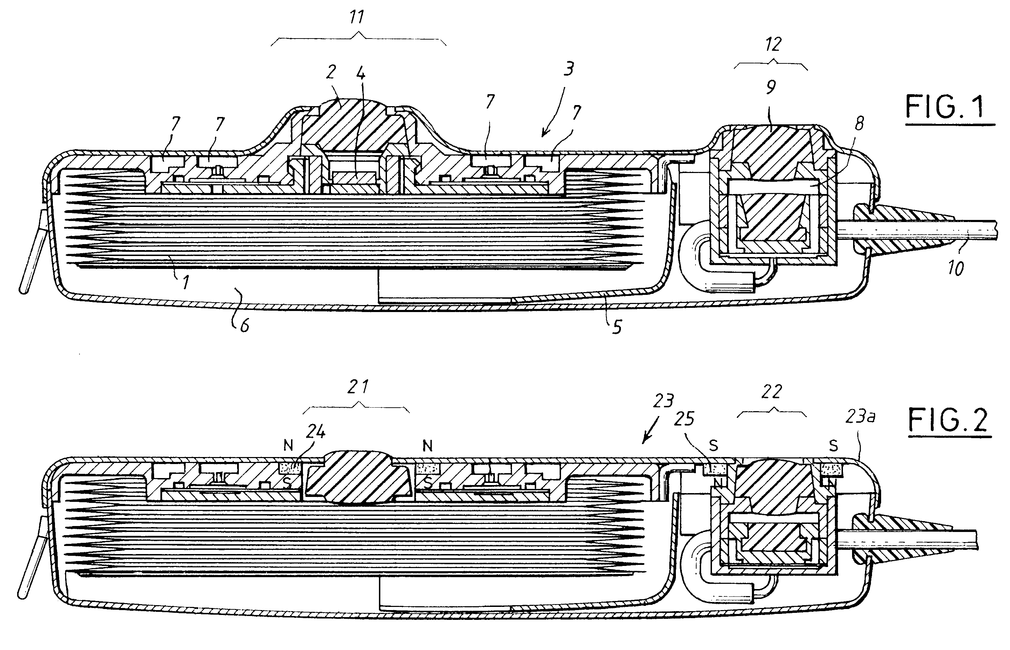

Figur 1 zeigt eine solche Infusionspumpe (3) im Schnitt. Sie besteht aus einem Balg (1), dessen Inhalt das Medikamentenreservoir bzw. -kammer bildet. Der Balg (1) des Medikamentenreservoir ist über einen Silikonstopfen (2), welcher einen wesentlichen Teil des Septums (11) bildet, zugänglich. Die Pumpe (3) wird im Bauchbereich unterhalb des Brustkorbs implantiert. Die Fassung des Septums (11) ist nach dem bekannten Stand der Technik als Domform ausgebildet. Dadurch ist es möglich, das Septum (11) später durch die Haut und das darunterliegende Fettgewebe hindurch zu ertasten. Nachdem der Arzt das Septum (11) ertastet hat, kann die Auffüllspritze angesetzt werden. Die Spritze wird durch Haut, Fettgewebe und Silikonstopfen (2) des Septums (11) gestochen. Ein Nadelstop (4) dient als Anschlag. Der Balg (1) der Medikamentenkammer (das Innere des Faltenbalges) kann auf diese Weise gefüllt werden. Der Balg (1) expandiert. Die Außenseite des Balges (1) liegt in einer druckdichten Dose (5), in der sich ein Gas (6) befindet, welches einen vom Volumen unabhängigen, konstanten Gegendruck erzeugt. Dieser Gegendruck versucht nun seinerseits das Medikament aus dem als Vorratskammer dienenden Balg (1) wieder auszutreiben. Der Fluß des ausströmenden Medikaments wird über eine Drosselstrecke (7) begrenzt, so daß sich eine konstante Flußrate einstellt. Das Medikament wird über eine Kammer (8), die über ein zusätzliches Bolusseptum (12) zugänglich ist, geführt und gelangt von dort aus über den Katheter (10) aus der Pumpe (3) heraus, an den Wirkort im Körper des Patienten. Über das Bolusseptum (12) mit dessen Silikonstopfen (9) können direkt Medikamente oder ein Kontrastmittel in den Katheter (10) und damit an den Wirkort eingespritzt werden, unter Umgehung der eigentlichen Pumpe (3). Auch das Bolusseptum (12) ist nach dem bekannten Stand der Technik domförmig ausgeführt und wird ertastet.

Das Ertasten der Septa (11, 12) bereitet bei adipösen (d.h. fettleibigen) Patienten Probleme. Die Lokalisierung ist schwierig und fast immer mit einer gewissen Unsicherheit behaftet. Wenn das Septum (11, 12) nicht getroffen wird, stößt die Injektionsnadel auf das Metallgehäuse der Pumpe (3) und kann verbiegen. Bei nochmaliger Punktierung hat sich durch den Fehlversuch die Nadelgeometrie verändert. Dies kann zu einer Beschädigung des Septums (11, 12) führen, was unbedingt vermieden werden muß. Die Septa (11, 12) müssen eine ausreichende Dichtwirkung aufweisen, um die unter Überdruck stehenden Medikamentenkammern (1, 8) sicher abzuschließen. Das heißt, der Silikonstopfen (2, 9) muß sich nach dem Herausziehen der Auffüllnadel selbständig wieder schließen.

Bei immer kleiner werdenden Pumpen (3) rückten beide Septa (11, 12) räumlich zwangsläufig immer enger zusammen. Neben der unzureichenden Ertastbarkeit kommt erschwerend eine mögliche Verwechslung beider Septa (11, 12) hinzu.

Für die Lokalisierung der Septa (11, 12) gibt es heutzutage mechanische Hilfen in Form von Schablonen. Diese Schablonen haben in etwa Konturen wie die Gehäuseform der Pumpe (3), werden als Leere auf die Haut aufgelegt und dienen als Orientierungshilfe zur Auffindung der Septa (11, 12). Die Verwendung derartiger Schablonen kann nur eine grobe Hilfestellung sein.

Es ist die Aufgabe der Erfindung, eine Infusionspumpe zu realisieren, bei welcher das Auffüllen des Vorratsbehälters für das zu verabreichende Medikament durch die an dem Vorratsbehälter angebrachte Einstichstelle (d.h. Auffüllseptum oder für ein vorhandenes Bolusseptum) sicher und problemlos erfolgen kann.

Diese Aufgabe wird erfindungsgemäß durch den kennzeichnenden Teil des ersten Patentanspruchs gelöst.

Es ist für den Tragekomfort des Patienten sehr vorteilhaft, wenn die vorgesehenen Einstichstelle in das Innere der Infusionspumpe flach gestaltet sind und mit der Oberfläche der Infusionspumpe formschlüssig abschließen. Die Ortbarkeit der Einstichstellen kann dann vorteilhafterweise durch einen axial magnetisierten Magneten sichergestellt werden, der konzentrisch zur Einstichstelle angeordnet ist, da dann deren Lage durch eine einfache Messung des Magnetfeldes ermittelt werden kann.

Die vorteilhafte Gestaltung dieser Lokalisierungseinrichtung ist in den Patentansprüchen und insbesondere in den Figurenbeschreibungen beschrieben.

Die Erfindung wird im folgenden anhand der Ausführungsbeispiele in den Zeichnungen Fig. 1 bis Fig. 3 näher erläutert. Dabei zeigen:
- Fig. 1: eine erste Infusionspumpe gemäß dem Stand der Technik;
- Fig. 2: eine zweite Infusionspumpe gemäß der Erfindung;
- Fig. 3: eine Detaildarstellung des Septa der Infusionspumpe aus Fig. 2 und der dortigen Verhältnisse;
- Fig. 4: eine Ausicht auf die Lokalisierungsgerät für die Infusionspumpe aus Fig. 2; und
- Fig. 5: der Schaltungsaufbau der Elektronik des Lokalisierungsgerätes aus Fig. 4.

Die in der Figur 1 dargestellte Infusionspumpe (3) wurde bereits vorab zum Stand der Technik beschrieben. In Fig.2 ist nun eine erfindungsgemäße Infusionspumpe dargestellt. Dabei ist die Infusionspumpe (23) in Fig. 2 im wesentlichen genauso aufgebaut wie die Infusionspumpe (3) in Fig. 1.

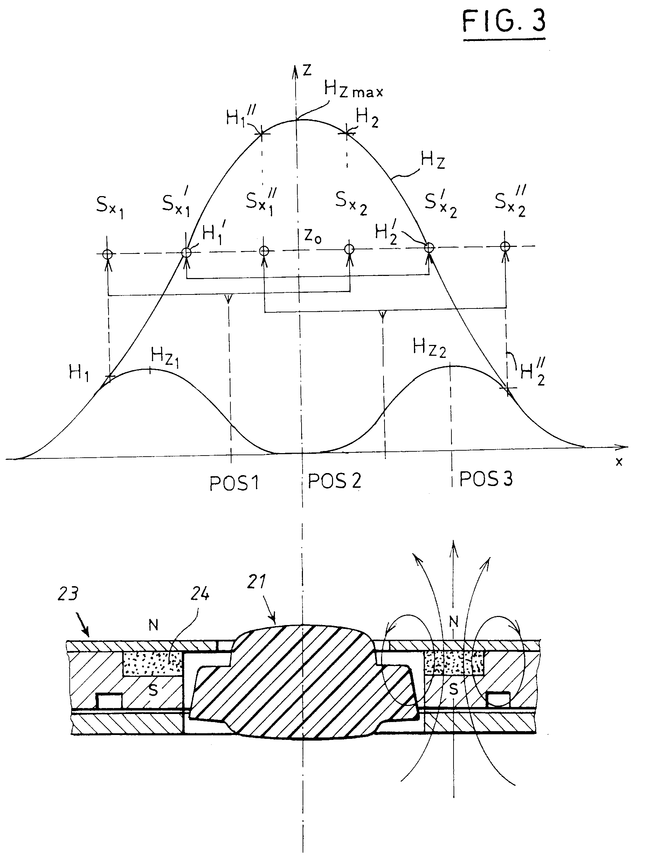

Der einzige Unterschied ist in der Ausführung des Auffüllseptums (21) und des Bolusseptums (22) zu finden. Im Gegensatz zu dem Auffüllseptum (11) und dem Bolusseptum (12) in Fig.1 schließen die Septa (21, 22) in Fig. 2 mit der Oberfläche der Infusionspumpe (23) ohne eine Erhebung ab. Der Arzt, welcher die Septa (21, 22) sucht, um in sie mit einer Infusionsnadel (in der Figur nicht eingezeichnet) hineinzustechen, kann diese nicht ertasten. Da aber die glatte Oberflache des Gehäuses (23a) der Infusionspumpe (23) für den Patienten, in welchem die Infusionspumpe (23) implantiert wurde, sehr angenehm ist (da die erhabenen Septa (11, 12 in Fig. 1) bei einer Bewegung Schmerzen verursachen können), sendet die Infusionspumpe (23) von außerhalb des Körpers meßbare magnetische Felder aus. Diese Felder werden von einem um die Septa (21, 22) angebrachten ringförmigen Permanentmagneten (24, 25) erzeugt, wobei die Magnetisierungsrichtung der beiden Permanentmagneten (24, 25) einander entgegengesetzt ist. Die genaue Beschreibung der Art und Weise, wie man mittels der beiden Permanentmagnete (24,25) um die Septa (21, 22) die Septa (21, 22) lokalisieren kann, erfolgt im Zusammenhang mit Fig. 3.

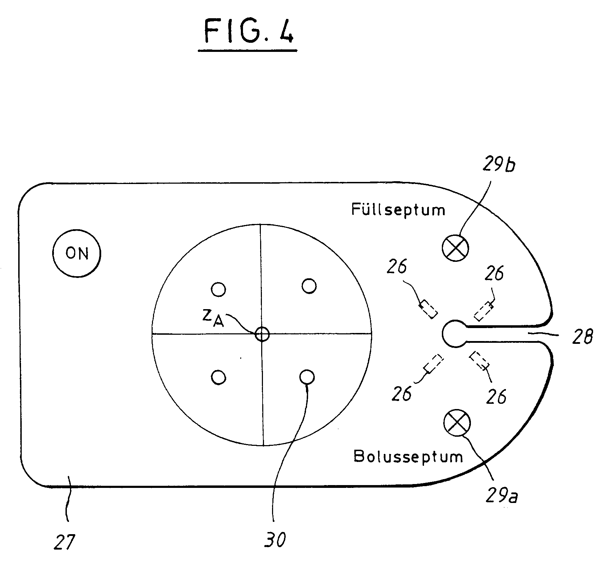

Durch den Einbau der zwei ringförmigen Permanentmagnete (24, 25) ist es dennoch möglich, die Septa (21, 22) von außerhalb des Patientenkörpers zu orten, und zwar durch Detektion der von den Septa (21, 22) ausgehenden magnetischen Felder, die extrakorporal mit einer geeigneten Ortungseinrichtung (27 in Fig.4) meßbar sind.

In den Figuren 3, 4 und 5 ist nun eine Lokalisationseinrichtung (27) detailliert dargestellt, welche dem Arzt die Suche nach dem Füllseptum (21) oder dem Bolusseptum (22) erleichtert und die Sicherheit wesentlich erhöht.

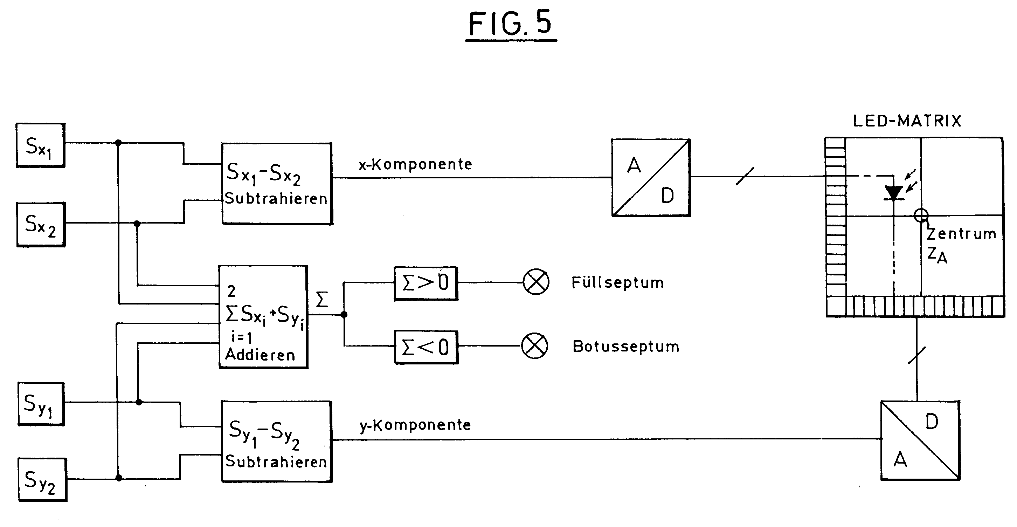

Der Permanentmagnet (24, 25) ist axial magnetisiert. Das Streufeld des Permanentmagneten (24,25) ragt aus der Infusionspumpe (23) heraus und ist in seiner Intensität räumlich zentrisch um den Mittelpunkt des betreffenden Septums (21, 22) verteilt. Dieses Magnetfeld und dessen Orientierung kann meßtechnisch erfaßt werden. Dazu legt man auf die Haut des Patienten mit implantierter Infusionspumpe (23) eine Art elektronische Leere (27, siehe Fig. 4 und 5) auf die Bauchdecke und kann diese durch Messung des Magnetfeldes mit magnetfeldempfindlichen Sensoren (26) (Sx_{1/2}/Sy_{1/2}) (z.B. mittels Hallfeldsensoren) in Position bringen. Der qualitative Feldverlauf ist in der Fig. 3 oberhalb des Septums (21) für ein Sensorpaar (26)(Sx_{1/2}) dargestellt. Die beiden lokalen Feldstärkenmaxima H_{z1}, H_{z2} verschmelzen mit größer werdendem Abstand Z zu einem Feldstärkenmaximum (H_{z,max.}) direkt über der Mitte des Septums (21). Benutzt man z.B. vier um jeweils 90° versetzt angeordnete magnetfeldempfindliche Sensoren (26), dann kann die Leere (27) auf maximale Feldstärke eingestellt werden, was der Mitte des Septums (21) entspricht.

Je nach Orientierung (x und Abstand z0) des Sensorpaares (26) Sx_{1/2} ergibt sich eine unterschiedliche Ausgangsspannung:
Pos. 1 U = const.*(H1-H2) : < 0 Verschieberichtung rechts
Pos. 2 U = const.*(H1'-H2') : = 0 mittig
Pos. 3 U = const.*(H1''-H2''): >0 Verschieberichtung links
Durch die Differenzmessung ist es damit möglich, die Richtung anzuzeigen, in die die Leere (27) zum Zentrieren verschoben werden muß. Die Sensoren (26)Sx_{1/2} messen die x-Komponente des Verschiebevektors. Die y-Komponente wird mit dem Sensorpaar (26) Sy_{1/2} gemessen. Durch die Wahl der Magnetisierungsrichtung ist es außerdem möglich, zwischen den zwei Septa (21, 22) zu unterscheiden. Je nachdem, ob beispielsweise der Südpol innenliegend oder außenliegend gewählt wird, kann man aufgrund der Feldrichtung entscheiden, ob es sich um das Auffüll- oder das Bolusseptum (21, 22) handelt.

Die Sensoren (26) Sx_{1/2} und Sy_{1/2} sind paarweise diagonal (Fig. 4) angeordnet. Die diagonale Anordnung erlaubt es, in das Gehäuse der Ortungseinrichtung (27) einen Schlitz (28) einzubringen. Nachdem das Septum (21, 22) gefunden worden ist, kann die Haut mit der Kanüle punktiert werden. Das geschlitzte Gehäuse der Leere (27) erlaubt die Entfernung der Ortungseinrichtung (27) bei eingestochener Kanüle.

In Fig. 5 ist der grundsätzliche Schaltungsaufbau der Elektronik als Blockschaltbild dargestellt. Durch Subtraktion der beiden paarweise zueinadergehörenden Sensorsignale Sx_{1/2} bzw. Sy_{1/2} der in der x-Achse bzw. y-Achse angeordneten Sensoren (26) in jeweils einem Subtrahierer erhält man die x- und die y-Komponente des Verschiebevektors. Der Verschiebevektor gelangt dann auf einen A/D-Wandler und wird mittels einer LED-Matrix (30) angezeigt, mit welcher der A/D-Wandler verbunden ist. Die Ansteuerung erfolgt direkt durch die A/D-Wandler der Komponentensignale. Die jeweils aufleuchtende LED der LED-Matrix (30) gibt die relative Lage zum Zentrum an und zeigt daher dem Benutzer, in welche Richtung die Leere (27) verschoben werden muß. Leuchtet die mittlere LED ZA in der Anzeige (LED-Matrix (30)) auf, dann ist das Einstichzentrum Z₂ (Fig. 4) in Deckung mit dem Zentrum des Septums (21, 22).

Zur Unterscheidung der Septa (21, 22) werden die Sensorsignale Sx_{1/2} und Sy_{1/2} der Sensoren (26) zusätzlich auf einen Addierer gegeben (Fig. 5). Das Summensignal wird mit zwei Komparatoren ausgewertet. Je nach Polarität werden unterschiedliche LEDs (29a, 29b) angesteuert, die anzeigen, ob es sich um ein Bolus- oder Auffüllseptum (21, 22) handelt.

Selbstverständlich können die Magnete (24, 25) auch durch geeignete andere Vorrichtungen ersetzt werden, welche extrakorporal detektiert werden können. Diese Vorrichtungen brauchen auch nicht immer aktiv sein, sondern können auch erst durch ein extrakorporales Signal aktiviert werden.

## Patentansprüche

1. Infusionspumpe zur Implantation in den Körper eines Patienten zur medikamentösen Behandlung des Patienten, welches mindestens eine Einstichstelle zum Auffüllen eines Vorratsbehälter zum Speichern eines gewählten Medikaments, eine flüssigkeitsfördernde Einrichtung zum Transport des Medikaments vom Vorratsbehälter und mindestens einer Vorrichtung zum begrenzten Einleiten des aus dem Vorratsbehälter kommenden, in diesem gespeicherten, ausgewählten Medikaments in den Körper aufweist, dadurch gekennzeichnet, daß um oder an mindestens einer der vorgesehenen Einstichstellen (21, 22) in die Infusionspumpe (23) mindestens eine extrakorporal detektierbare Einrichtung (24,25) angeordnet ist, so daß zur Injektion in diese Einstichstelle (21, 22) deren Lage extrakorporal bestimmt werden kann.

2. Infusionspumpe nach Anspruch 1, dadurch gekennzeichnet, daß um oder an mindestens einer der vorgesehenen Einstichstellen (21, 22) in die Infusionspumpe (23) mindestens ein axial magnetisierter Magnet (24, 25) angeordnet ist, so daß zur Injektion in diese Einstichstelle (21, 22) deren Lage durch Magnetfeldmessung bestimmt werden kann.

3. Infusionspumpe nach einem der Ansprüche 1 oder 2, dadurch gekennzeichnet, daß zur Zentrierung für eine Injektion in die Infusionspumpe (21, 22) in einer Lagebestimmungseinrichtung (279 mindestens zwei differentiell messende Magnetfeldsensoren (26) angeordnet sind.

4. Infusionspumpe nach einem der Ansprüche 2 oder 3, dadurch gekennzeichnet, daß die Magnetisierung der Magnete (24, 25) um oder an den zueinander am nächsten gelegenen Einstichstellen (21, 22) (Auffüll- oder Bolusseptum) unterscheidbar ist.

5. Infusionspumpe nach einem der Ansprüche 2-4, dadurch gekennzeichnet, daß die Einstichstelle (21, 22) (Auffüll- oder Bolusseptum) mit Magnet (24, 25) mit der Oberfläche der Infusionspumpe (23) bündig abschließend ist.

6. Infusionspumpe nach einem der Ansprüche 1-5, dadurch gekennzeichnet, daß der Magnet (24, 25) ein permanentmagnetischer Ring ist.
